(19) Europäisches Patentamt / European Patent Office / Office européen des brevets

(11) EP 2 923 577 A1

(12) **EUROPEAN PATENT APPLICATION**
published in accordance with Art. 153(4) EPC

(43) Date of publication:
30.09.2015 Bulletin 2015/40

(51) Int Cl.:
*A01N 63/02* (2006.01)    *A01N 65/34* (2009.01)
*A61K 8/64* (2006.01)    *A61K 8/97* (2006.01)
*A61Q 11/00* (2006.01)

(21) Application number: 13854691.6

(22) Date of filing: 08.11.2013

(86) International application number:
PCT/JP2013/080192

(87) International publication number:
WO 2014/077188 (22.05.2014 Gazette 2014/21)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 14.11.2012 JP 2012249988

(71) Applicants:
• Eco Friendly Institute Limited
Chikushino-shi, Fukuoka 818-0013 (JP)

• Trife.Inc.
Yokohama-shi, Kanagawa225-0023 (JP)

(72) Inventor: NAGATOSHI Kohei
Chikushino-shi
Fukuoka 818-0013 (JP)

(74) Representative: Godemeyer Blum Lenze
Patentanwälte
Partnerschaft mbB - werkpatent
An den Gärten 7
51491 Overath (DE)

(54) **ANTIMICROBIAL COMPOSITION**

(57)    The present invention provides a highly safe antibacterial composition showing antibacterial activity against both Gram-positive bacteria and Gram-negative bacteria, of which flavor is not impaired. The antibacterial composition of the present invention contains nisin, and one or two or more kinds of plant-derived ingredients selected from the group consisting of juice, extract, and distillate of a *Rosaceae* plant, and a mixture of these. It is preferred that the *Rosaceae* plant is a plant belonging to the genus *Prunus*, it is more preferred that the plant belonging to the genus *Prunus* is *Prunus mume*, and it is particularly preferred that the plant-derived ingredient is *Prunus mume* fruit juice. The antibacterial composition of the present invention is useful especially as a composition for oral cavity.

[Fig. 1]

EP 2 923 577 A1

**Description**

Technical Field

The present invention relates to an antibacterial composition containing nisin as a sterilizing component and effective for both Gram-positive bacteria and Gram-negative bacteria.

Background Art

Nisin is a kind of bacteriocin, and is a polycyclic antibacterial peptide consisting of 34 amino acid residues produced by *Lactococcus lactis* isolated from cheese. Nisin is a water-soluble polypeptide, and shows growth-suppressing effect against Gram-positive bacteria including acetic acid bacteria even at a low concentration as low as about 1 ppb, and it shows a large antibacterial spectrum unlike other bacteriocins, which show antibacterial activity against only close species. Nisin is approved as a food additive in many countries including Japan, and is applied to food preservatives, various kinds of antibacterial agents, and so forth.

Since nisin shows antibacterial activity against Gram-positive bacteria, it is expected that it is effective for disinfection of *Streptococcus mutans,* which is a causative bacterium of caries. Therefore, there is expected application of nisin for safer mouth-rinsing solution (mouthwash) that replaces the conventional mouth-rinsing solutions containing sodium laurylsulfate, propylene glycol etc., for which toxicity is pointed out. However, since nisin cannot show antibacterial activity against Gram-negative bacteria by itself, it is not effective for disinfection of causative bacteria of periodontal disease such as *Porphyromonas gingivalis,* which is a Gram-negative bacterium. It is known that an antibacterial composition showing antibacterial activity against both Gram-positive bacteria and Gram-negative bacteria can be obtained by combining a chelating agent such as EDTA and nisin (refer to, for example, Patent document 1, Non-patent documents 1 and 2). There has also been proposed a bactericide comprising fumaric acid, which shows bactericidal action against Gram-negative bacteria, nisin, which shows bactericidal action against Gram-positive bacteria, lactic acid (for example, 0.8% (w/v)), and one of phosphoric acid and citric acid, in which the concentration of fumaric acid is 0.03 to 0.1% (w/v), and the concentration of nisin is 1 to 100 ppm (refer to Patent document 2).

Prior art references

Patent documents

Patent document 1: Japanese Patent Unexamined Publication (Kohyo) No. 3-500051
Patent document 2: Japanese Patent No. 4309822

Non-patent documents

Non-patent document 1: Applied and Environmental Microbiology, 58(5), pp. 1786-88, 1992
Non-patent document 2: International Journal of Food Microbiology, 21, pp.305-314, 1994

Summary of the Invention

Object to be Achieved by the Invention

However, EDTA has toxicity to human bodies, and therefore it is not preferable to use EDTA as a raw material of antibacterial compositions used for human bodies such as those for oral cavity care from the viewpoint of safety. Further, since the antibacterial agent described in Patent document 2 contains organic acids such as lactic acid in addition to fumaric acid, they degrade flavors such as scent and taste, and thus it has a problem as application to antibacterial compositions used for human bodies such as those for oral cavity care.

The present invention was accomplished in view of such a situation as described above, and an object thereof is to provide a highly safe antibacterial composition showing antibacterial activity against both Gram-positive bacteria and Gram-negative bacteria, of which flavor is not impaired.

Means for Achieving the Object

The present invention achieves the aforementioned object by providing an antibacterial composition containing nisin, and one or two or more kinds of plant-derived ingredients selected from the group consisting of juice, extract, and distillate of a *Rosaceae* plants, and a mixture of these.

In the antibacterial composition of the present invention, it is preferred that the *Rosaceae* plant(s) is a plant belonging to the genus *Prunus*, it is more preferred that the plant belonging to the genus *Prunus* is *Prunus mume* (Japanese apricot), and it is particularly preferred that the plant-derived ingredient is *Prunus mume* fruit juice.

In the antibacterial composition of the present invention, it is preferred that content of nisin in the antibacterial composition is 0.1 μg/mL to 10 mg/mL, and content of the plant-derived ingredient (*Prunus mume* fruit juice) in the antibacterial composition is 1 to 200 mg/mL. It is preferred that the antibacterial composition of the present invention is a composition for oral cavity.

Effect of the Invention

According to the present invention, by using a mixture of nisin and a *Rosaceae* plant-derived ingredient, there can be provided an antibacterial composition that shows antibacterial activity also against, besides Gram-positive bacteria, Gram-negative bacteria, against which nisin cannot show antibacterial activity by itself. Moreover, since the antibacterial composition of the present invention does not contain compounds of which use in a large amount may be toxic to human bodies, such as chelating agents and sodium laurylsulfate, as essential ingredients, it is extremely safe.

Brief Explanation of the Drawings

[Fig. 1] Fig. 1 is a graph showing the antibacterial effects of *Prunus mume* extract, nisin, and nisin + *Prunus mume* extract against *Porphyromonas gingivalis* determined in Example 1.

[Fig. 2] Fig. 2 is a graph showing the antibacterial effects of *Prunus mume* extract, nisin, and nisin + *Prunus mume* extract against *Porphyromonas gingivalis* determined in Example 2.

[Fig. 3] Fig. 3 is a graph showing the antibacterial effect of nisin against *Escherichia coli* determined in Example 3.

[Fig. 4] Fig. 4 is a graph showing the antibacterial effects of *Prunus mume* extract, and nisin + *Prunus mume* extract against *Escherichia coli* determined in Example 3.

[Fig. 5] Fig. 5 is a graph showing the antibacterial effects of *Prunus mume* extract, nisin, and nisin + *Prunus mume* extract against *Escherichia coli* determined in Example 4.

Modes for Carrying out the Invention

Hereafter, embodiments of the present invention will be explained for better understanding of the present invention.

The antibacterial composition according to an embodiment of the present invention (henceforth also referred to simply as "the antibacterial composition" or "composition") contains nisin and one or two or more kinds of plant-derived ingredients selected from the group consisting of juice, extract, and distillate of a *Rosaceae* plant, and a mixture of these. The antibacterial composition of the present invention is especially useful as a composition for oral cavity.

(1) Nisin

Nisin is a kind of bacteriocin, and is a low molecular weight protein consisting of 34 amino acid residues containing lanthionine, which is an abnormal amino acid. Type of nisin usable for preparing the antibacterial composition is not particularly limited, and examples include, for example, nisin A and nisin Z. The structures of nisin A and bisin Z are similar to each other. They are different only in that the 27th amino acid from the N-terminus in the polypeptide chain of nisin A is histidine, whereas the same of nisin Z is asparagine.

Nisin can be obtained by culturing lactic acid bacteria by a known method, and performing purification. A purified product of nisin can be obtained by, for example, culturing lactic acid bacteria in the MRS medium (produced by Oxoid), then treating the culture supernatant with a synthetic adsorbent material such as Amberlite XAD-16 (produced by Sigma) to adsorb nisin on the adsorbent material, washing Amberlite with distilled water and 40% ethanol, then eluting nisin with 70% isopropyl alcohol containing 0.1% trifluoroacetic acid, and applying the eluted fraction to a cation exchange column (for example, SP-Sepharose FF, produced by GE Healthcare Bioscience). If needed, the product may be subjected to reverse phase chromatography to further improve the purification degree (refer to, for example, Biosci. Biotechnol. Biochem., 67(7), pp.1616-1619, 2003). A commercially available product (for example, Nisaplin (trademark, produced by Danisco)) may also be used. Nisaplin is a mixture of nisin derived from *Lactococcus lactis* subsp. *lactis,* and sodium chloride containing non-fat milk or saccharide medium-derived ingredients. If needed, the ingredients derived from the medium etc. may be removed by the aforementioned purification method to improve the purity.

Although content of nisin in the antibacterial composition is not particularly limited so long as desired antibacterial activity is exhibited, it is preferably 0.1 $\mu$g/mL to 10 mg/mL, more preferably 0.5 $\mu$g/mL to 1 mg/mL, still more preferably 1 to 250 $\mu$g/mL.

(2) Plant-derived ingredient

*Rosaceae* plants are dicotyledonous plants, and in various life forms, namely, for example, they are a tree or shrub, and a perennial herb or therophyte. They are distributed over all the continents except for the antarctic continent, but many of them are distributed especially over the warm temperate zone or the temperate zone of the northern hemisphere. They show various inflorescences, but many of them bear bisexal radially symmetric flowers.

Although the *Rosaceae* plant used for preparing the antibacterial composition is not particularly limited, examples include, for example, those belonging to genus *Agrimonia, Pyracantha, Spiraea, Potentilla, Fragaria, Geum, Duchesnea, Rubus, Sanguisorba, Rosa, Prunus, Crataegus, Eriobotrya, Chaenomeles,* or *Malus.* The *Rosaceae* plant is preferably a *Prunus* plant, and specific example thereof include almond (*Prunus dulcis*), peach (*Prunus persica*), prune (*Primus domestica*), Japanese apricot (*Prunus mume*), plum (*Primus salicina*), blackthorn (*Prunus spinosa*), apricot (*Primus vulgaris*), sweet cherry (*Primus avium*), Taiwan cherry (*Prunus campanulata*), sour cherry (*Prunus cerasus*), *Prunus jamasakura* Sieb. ex Koidz (*Prunus jamasakura*), *Prunus leveilleana, Prunus pendula,* Chinese cherry (*Prunus pseudocerasus*), Oshima cherry (*Prunus speciosa*), *Prunus verecunda, Prunus x mochizukiana,* Somei Yoshino (*Prunus x yedoensis*), downy cherry (*Primus tomentosa*), *Prunus avium* Mill, Japanese bird cherry (*Primus grayana*)*, Prunus buergeriana,* cherry laurel (*Primus laurocerasus*), *Prunus spinulosa,* and *Prunus zippeliana.*

As the plant-derived ingredient, juice, extract, and distillate of *Rosaceae* plants are used. Examples of part of *Rosaceae* plants include terrestrial parts including flower parts (whole flower or parts of flowers such as petal, calyx, pistil, and stamen), trunk parts (whole trunk or parts of trunk such as bark), leaf, seed, fruit (whole fruit including seed and parts of fruit such as pericarp and pulp), and so forth, and underground parts including root, underground stem, and bulb. Among these, arbitrary two or more may be used in combination.

Preferred examples of the plant-derived ingredient include fruit juice of *Prunus mume*, plum, apricot, or the like, and fruit juice of *Prunus mume*, which is a plant belonging to genus *Prunus*, is especially preferred. For squeezing juice from fruits or other parts, arbitrary methods can be used without any particular restriction. For squeezing juice, whole fruits including pericarps and seeds may be used, or fruits from whish such parts are eliminated may be used, and if needed, those subjected to a pretreatment such as cutting into strips and grinding may also be used. For separation of residue and juice, cloth, filter paper, ultrafiltration membrane, glass filter, or the like may be used, and such a separation method as centrifugal separation and decantation may also be used. The obtained juice may be concentrated in an appropriate degree by using arbitrary means and methods, and used as a concentrate or solid.

For the extraction or distillation, plant body of a *Rosaceae* plant or a part thereof may be used as it is, or a processed product thereof obtained by drying, cutting into strips, grinding, or the like may be used. Examples of the solvent used for obtaining the extract include, as polar solvents, water, organic solvents such as lower alcohols, higher alcohols, and ketones, and arbitrary mixtures of two or more kinds of these solvents. Examples of nonpolar solvents include petroleum ether, aliphatic hydrocarbons having 4 to 8 carbon atoms, halogenated hydrocarbons having 1 or 2 carbon atoms, aromatic hydrocarbons having 6 to 7 carbon atoms, and so forth. The extraction method is not particularly limited, and any of solid-liquid extraction methods such as the Soxhlet method, liquid-liquid extraction methods, and extraction methods using supercritical fluid, and so forth may be used.

The distillation method for obtaining distillate is not also particularly limited, and arbitrary methods including atmospheric distillation method, vacuum distillation method, and steam distillation method can be appropriately used.

The juice, extract, and distillate obtained as described above may be used as they are, or those subjected to further purification (arbitrary methods such as precision distillation, column chromatography, and recrystallization can be used) may be used, if needed. Concentrates of these may also be used. Arbitrary two or more kinds of them may also be used in combination.

The content of the plant-derived ingredient in the antibacterial composition is not particularly limited so long as the antibacterial activity against Gram-negative bacteria can be obtained, but it is preferably 1 to 200 mg/mL, more preferably 1.5 to 150 mg/mL, still more preferably 2 to 100 mg/mL, particularly preferably 2.5 to 50 mg/mL. When a concentrate of juice, extract, or distillate is used as the plant-derived ingredient, it is preferred that the content of the plant-derived ingredient in juice, extract, or distillate before concentration is within the aforementioned range.

The antibacterial composition has antibacterial effects against microorganisms (including both Gram-positive bacteria and Gram-negative bacteria) in the oral cavity that cause undesired diseases or conditions in the oral cavity, for example, diseases of teeth and gingiva such as caries and periodontal disease, bad smell and dental plaque formation as related symptoms or prodromes of the foregoing diseases or conditions. The antibacterial composition may be in the form of, for example, mouth-rinsing solution (mouthwash), mouth-rinsing spray (mouse spray), toothpaste, dental powder, tooth brushing cream, gel, chewing gum, gum containing liquid at the center (liquid center filled gum), mint agent, troche, film for oral cavity, or the like.

Besides such use for oral cavity care as mentioned above, the antibacterial composition can be used for products for infectious diseases caused by bacteria or for killing causative bacteria. For example, it can be used for face care products such as those for acne care, body care products such as body deodorants (for foot, underarm) and those for prevention of infants' impetigo, eye care products such as eye lotion and contact lens washing solution, and so forth.

The antibacterial composition of course may contain other arbitrary bases and additives usually used for antibacterial compositions of a desired form according to the form thereof. In the case of toothpaste, for example, it may contain, as additives, abrasives such as calcium hydrogenphosphate, calcium carbonate, and aluminum hydroxide, foraming agents such as sodium lauroylsarcosinate, sodium laurylsulfate, and sucrose fatty acid esters, moisturizers such as sorbitol, glycerin, and propylene glycol, binders such as xanthan gum, sodium arginate, and carboxymethylcellulose, sweetners such as xylitol, perfumes such as menthol, corrigents such as calcium lactate, auxiliaries such as ethanol, and pharmaceutically effective ingredients such as fluorides, dextranase, glucose oxidase, chlorhexidine, and lysozyme chloride.

In the case of mouth-rinsing solution, water such as purified water is used as a base, and there may be used moisturizers such as glycerin and sorbitol, sweetners such as hydrogenated starch and xylitol, solubilizers such as polyoxyethylene-polyoxypropylene block copolymers (Poloxamer etc.), binders such as hydroxyethylcellulose, perfumes such as peppermint and aloe vera sap, essential oils such as thymol, menthol, methyl salicylate (wintergreen oil), eucalyptol, carvacrol, camphor, anethole, carvone, eugenol, isoeugenol, limonene, osimen, n-decyl alcohol, citronel, $\alpha$-salpineol, methyl acetate, citronellyl acetate, methyl eugenol, cineol, linalool, ethyl linalool, safrola vanillin, spearmint oil, peppermint oil, lemon oil, orange oil, sage oil, rosemary oil, cinnamon oil, pimento oil, laurel oil, cedar leaf oil, gerianol, verbenone, anise oil, bay oil, benzaldehyde, bergamot oil, bitter almond, chlorothymol, cinnamic aldehyde, citronella oil, clove oil, coal tar, eucalyptus oil, guaiacol, lavender oil, mustard oil, phenol, phenyl salicylate, pine oil, pine needle oil, sassafras oil, spike lavender oil, storax, thyme oil, tolu balsam, turpentine oil, and clove oil, auxiliaries such as ethanol, lysozyme chloride, lactoferrin, and glucose oxidase, and corrigents such as calcium lactate.

Examples of microorganisms as the target of the antibacterial activity of the antibacterial composition include *Fusobacterium nucleatum*, *Prevotella intermedia*, *Actinomyces viscosus*, *Campylobacter rectus*, *Porphyromonas gingivalis*, *Streptococcus sanguis*, *Streptococcus mutans*, *Actinobacillus* bacteria, *Bacteroides* bacteria, *Capnocytophaga* bacteria, *Eikenella* bacteria, *Propionibacterium* bacteria, *Candida albicans*, and so forth, but are not limited to these.

Examples

Hereafter, examples performed for confirming the effect of the present invention will be explained.

In the following examples, nisin A is used as nisin, and a 5-fold concentrate of *Prunus mume* fruit juice (BX82, acidity 51%, henceforth referred to as "*Primus mume* extract") was used as the plant-derived ingredient.

Example 1: Antibacterial activity against *Porphyromonas gingivalis* (1)

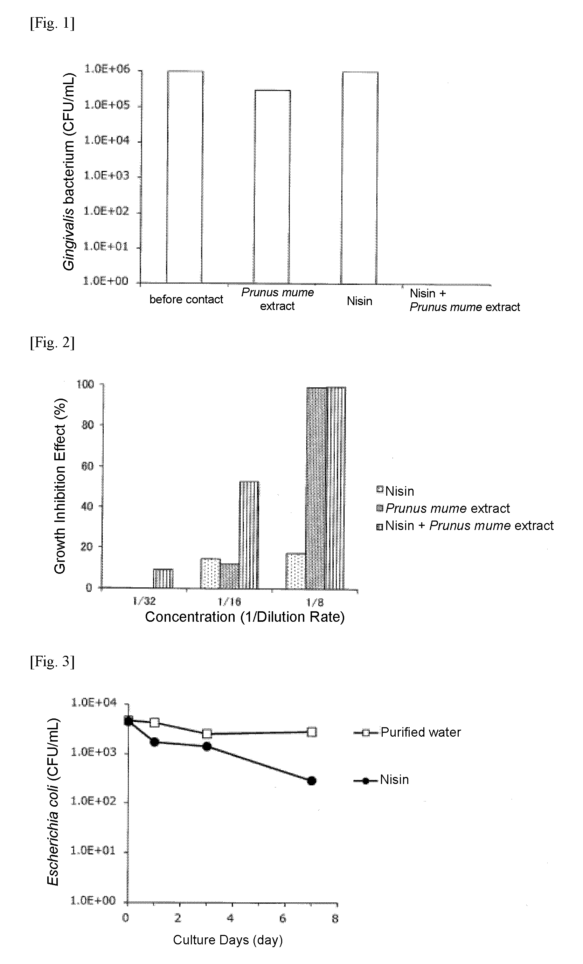

The *Porphyromonas gingivalis* ATCC 33277 strain, a causative bacterium of periodontal disease, was cultured to the logarithmic phase in Enriched Tryptic Soy Broth (e-TSB). The culture medium was diluted with a citrate buffer (pH 4.5) containing 0.8% NaCl to adjust the number of bacteria ($10^6$ CFU/mL). Test solutions were prepared by dilution with sterilized purified water so as to contain the 5-fold concentrate of *Prunus mume* extract (3 mg/mL, 15 mg/mL as the extract before concentration), nisin (150 $\mu$g/mL), or nisin (150 $\mu$g/mL) + 5-fold concentrate of *Primus mume* extract (3 mg/mL, 15 mg/mL as the extract before concentration). The diluted culture medium (100 $\mu$L) was added to each test solution (5 mL). After contact of the bacterium with the test solution for 1 minute at room temperature, the mixture (100 $\mu$L) was added to and mixed with the e-TSB medium (5 mL), and the mixture (100 $\mu$L) was immediately plated on a blood agar medium under an anaerobic condition. The bacterium was cultured for 5 days under an anaerobic condition, and then the number of the colonies formed on the plate was counted. The results were as shown in Table 1 mentioned below and Fig. 1. In Table 1, "CFU" means colony-forming unit (the same shall apply to the following descriptions).

[Table 1]

Table 1

| Sample | Number of bacteria (CFU/mL) | |
|---|---|---|
| | Before contact | After contact |
| *Primus mume* extract (3 mg/mL) | $1.0 \times 10^6$ | $3.0 \times 10^5$ |
| Nisin (150 $\mu$g/mL) | $1.0 \times 10^6$ | $1.0 \times 10^6$ |
| Nisin (150 $\mu$g/mL) + *Primus mume* extract (3 mg/mL) | $1.0 \times 10^6$ | 0 |

As seen from the results shown in Table 1 and Fig. 1, it was confirmed that nisin alone did not show any antibacterial activity at all against *Porphyromonas gingivalis,* which is a Gram-negative bacterium, and the *Prunus mume* extract alone also could not show sufficient antibacterial activity against the same, but extremely high antibacterial activity could be obtained with the combination of the both.

Example 2: Antibacterial activity against *Porphyromonas gingivalis* (2)

The *Porphyromonas gingivalis* W83 strain, a causative bacterium of periodontal disease, was cultured to the logarithmic phase in the brain heart infusion medium containing hemin (5 mg/L) and metidione (1 mg/L). The culture medium was diluted with a citrate buffer containing 0.8% NaCl to adjust the number of bacteria ($10^6$ CFU/mL). Test solutions were prepared by dilution with sterilized purified water so as to contain the 5-fold concentrate of *Primus mume* extract (10 mg/mL, 50 mg/mL as the extract before concentration), nisin (20 $\mu$g/mL), or the 5-fold concentrate of *Prunus mume* extract (10 mg/mL, 50 mg/mL as the extract before concentration) + nisin (50 $\mu$g/mL), and serially diluted 2-fold on a 96-well plate. To each well (containing 50 $\mu$L of test solution), the brain heart infusion medium (140 $\mu$L) containing hemin (5 mg/L) and metidione (1 mg/L) and the cell suspension (10 $\mu$L) were added. After culture for 48 hours, turbidity of the culture was measured at 600 nm. For evaluation, growth inhibition effect (%) was calculated according to the equation:

$$\text{Growth inhibition effect (\%)} = (1 - (\text{Turbidity observed with test solution})/(\text{Turbidity observed with sterilized water})) \times 100.$$

The results are shown in Fig. 2.

As seen from the results shown in Fig. 2, the growth inhibition effect of nisin alone against *Porphyromonas gingivalis,* which is a Gram-negative bacterium, was extremely low, and it did not show any growth inhibition effect at all at the 1/32 concentration (32-fold dilution). The *Prunus mume* extract alone also showed extremely low growth inhibition effect at the 1/16 concentration (16-fold dilution), and did not show any growth inhibition effect at all at the 1/32 concentration (32-fold dilution). In contrast, it was confirmed that the combination of the both showed the growth inhibition effect at a constantly maintained level at the 1/16 concentration (16-fold dilution), and also showed the growth inhibition effect even at the 1/32 concentration (32-fold dilution).

Example 3: Antibacterial activity against *Escherichia coli* (1)

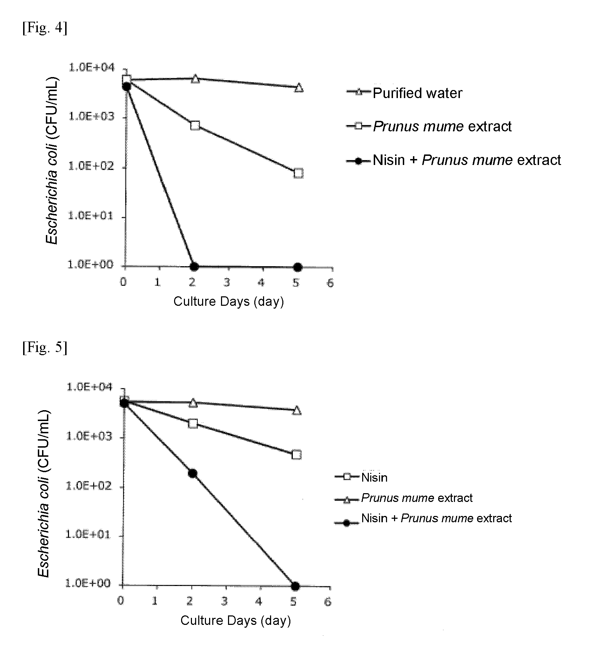

The *Escherichia coli* NBRC 3301 strain, which is a Gram-negative bacterium, was cultured to the logarithmic phase in Tryptic Soy Broth (TSB). The culture medium was diluted with sterilized purified water to adjust the number of bacteria ($10^3$ to $10^4$ CFU/mL). Test solutions were prepared by dilution with sterilized purified water so as to contain nisin (10 $\mu$g/mL), the 5-fold concentrate of *Primus mume* extract (3 mg/mL, 15 mg/mL as the extract before concentration), or nisin (10 $\mu$g/mL) + 5-fold concentrate of *Prunus mume* extract (3 mg/mL, 15 mg/mL as the extract before concentration). The diluted culture medium (10 $\mu$L) was added to each test solution (1 mL). After contact of the bacterium with the test solution at room temperature for a predetermined number of days (0 to 7 days), the mixture (100 $\mu$L) was plated on TBS agar medium. The bacterium was cultured for 48 hours, and then the number of the colonies formed on the plate was counted. The results are shown in Tables 2 and 3 mentioned below and Figs. 3 and 4. In Tables 2 and 3, "D+n" means the number of days (n days) for the contact of the diluted culture medium and the test solution (the same shall apply to the following descriptions).

[Table 2]

Table 2

| Sample | Number of bacteria (CFU/mL) | | | |
|---|---|---|---|---|
| | D+0 | D+1 | D+3 | D+7 |
| Control (purified water) | 4,720 | 4,290 | 2,550 | 2,830 |
| + 10 µg/mL Nisin | 4,540 | 1,740 | 1,430 | 290 |

[Table 3]

Table 3

| Sample | Number of bacteria (CFU/mL) | | |
|---|---|---|---|
| | D+0 | D+2 | D+5 |
| Control (purified water) | 6,110 | 6,610 | 4,470 |
| + 3 mg/mL *Primus mume* extract | 6,080 | 720 | 81 |
| + 10 µg/mL Nisin | 4,420 | 0 | 0 |

As seen from the results shown in Tables 2 and 3 and Figs. 3 and 4, it was confirmed that extremely higher antibacterial activity was obtained with the combination of nisin and the *Prunus mume* extract compared with that obtained with each alone.

Example 4: Antibacterial activity against *Escherichia coli* (2)

The *Escherichia coli* NBRC 3301 strain, which is a Gram-negative bacterium, was cultured to the logarithmic phase in Tryptic Soy Broth (TSB). The culture medium was diluted with sterilized purified water to adjust the number of bacteria ($10^3$ to $10^4$ CFU/mL). The compositions of the samples (Nos. 1 to 3) used as test solutions were as shown in Table 4 mentioned below. The diluted culture medium (10 µL) was added to each test solution (1 mL). After contact of the bacterium with the test solution at room temperature for a predetermined number of days (0 to 5 days), the mixture (100 µL) was plated on TBS agar medium. The bacterium was cultured for 48 hours, and then the number of the colonies formed on the plate was counted. The results are shown in Table 5 mentioned below and Fig. 5.

[Table 4]

Table 4

| Sample | No. 1 | No. 2 | No. 3 |
|---|---|---|---|
| Purified water | 95.0 | 98.7 | 94.7 |
| Xanthan gum | 1.0 | 1.0 | 1.0 |
| 5-Fold concentrated *Prunus mume* extract | | 0.3 (3 mg/mL) | 0.3 (3 mg/mL) |
| Nisin A | 4.0 (20 µg/mL) | | 4.0 (20 µg/mL) |
| Unit: % | | | |

[Table 5]

Table 5

| Sample | Number of bacteria (CFU/mL) | | |
|---|---|---|---|
| | D+0 | D+2 | D+5 |
| No. 1 (nisin) | 5,600 | 2,000 | 490 |
| No. 2 (*Prunus mume* extract) | 5,400 | 5,290 | 3,830 |
| No. 3 (nisin + *Prunus mume* extract) | 5,000 | 200 | 0 |

As seen from the results shown in Tables 5 and Fig. 5, it was confirmed that, also when the compositions in the form of gel were used, extremely higher antibacterial activity was obtained with the combination of nisin and the *Prunus mume* extract compared with that obtained with each alone, as demonstrated in Example 2.

**Claims**

1. An antibacterial composition containing:

    nisin, and
    one or two or more kinds of plant-derived ingredients selected from the group consisting of juice, extract, and distillate of a *Rosaceae* plant, and a mixture of these.

2. The antibacterial composition according to claim 1, wherein the *Rosaceae* plant is a plant belonging to the genus *Prunus*.

3. The antibacterial composition according to claim 2, wherein the plant belonging to the genus *Prunus* is *Prunus mume*.

4. The antibacterial composition according to claim 3, wherein the plant-derived ingredient is *Prunus mume* fruit juice.

5. The antibacterial composition according to claim 4, wherein content of nisin in the antibacterial composition is 0.1 $\mu$g/mL to 10 mg/mL, and content of the *Prunus mume* fruit juice in the antibacterial composition is 1 to 200 mg/mL.

6. The antibacterial composition according to any one of claims 1 to 3, wherein content of nisin in the antibacterial composition is 0.1 $\mu$g/mL to 10 mg/mL, and content of the plant-derived ingredient in the antibacterial composition is 1 to 200 mg/mL.

7. The antibacterial composition according to any one of claims 1 to 6, which is a composition for oral cavity.

[Fig. 1]

[Fig. 2]

[Fig. 3]

[Fig. 4]

[Fig. 5]

<table>
<tr><td colspan="2" align="center">**INTERNATIONAL SEARCH REPORT**</td><td colspan="2">International application No.<br>PCT/JP2013/080192</td></tr>
</table>

A. CLASSIFICATION OF SUBJECT MATTER
*A01N63/02*(2006.01)i, *A01N65/34*(2009.01)i, *A61K8/64*(2006.01)i, *A61K8/97*
(2006.01)i, *A61Q11/00*(2006.01)i

According to International Patent Classification (IPC) or to both national classification and IPC

B. FIELDS SEARCHED

Minimum documentation searched (classification system followed by classification symbols)
A01N63/02, A01N65/34, A61K8/64, A61K8/97, A61Q11/00

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/MEDLINE/WPIDS/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | CN 102578682 A (JIANGSU AGRIC SCI INST), 18 July 2012 (18.07.2012), claims (Family: none) | 1-2 |
| Y | JP 5-320017 A (Unilever N.V.), 03 December 1993 (03.12.1993), claims; examples 1 to 7 & EP 466244 A1 & CA 2046452 A | 1-7 |
| Y | JP 3-500051 A (Public Health Research Institute of the City of New York), 10 January 1991 (10.01.1991), claims; page 2, lower right column, line 21 to page 3, upper left column, line 6 & WO 89/12399 A1 & EP 382814 B1 & US 5135910 A & DE 68913189 A | 1-7 |

| ☒ | Further documents are listed in the continuation of Box C. | ☐ | See patent family annex. |

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>03 February, 2014 (03.02.14) | Date of mailing of the international search report<br>10 February, 2014 (10.02.14) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2013/080192 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2006-273817 A  (Naris Cosmetics Co., Ltd.),<br>12 October 2006 (12.10.2006),<br>paragraph [0002]<br>(Family: none) | 1-7 |
| Y | JP 5-161469 A  (Kozaburo SHIBA),<br>29 June 1993 (29.06.1993),<br>paragraph [0003]<br>(Family: none) | 1-7 |
| Y | JP 8-504404 A  (Smith Kline Beecham PLC),<br>14 May 1996 (14.05.1996),<br>claims<br>& WO 94/12150 A1          & EP 670711 A1<br>& GB 9224598 A           & DE 69323558 A<br>& CN 1101254 A | 1-7 |
| P,X | CN 103099290 A  (UNIV ZHEJIANG OCEAN),<br>15 May 2013 (15.05.2013),<br>claims<br>(Family: none) | 1-2,6 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3500051 A

- JP 4309822 B

**Non-patent literature cited in the description**

- *Applied and Environmental Microbiology,* 1992, vol. 58 (5), 1786-88
- *International Journal of Food Microbiology,* 1994, vol. 21, 305-314

- *Biosci. Biotechnol. Biochem.,* 2003, vol. 67 (7), 1616-1619